# EUROPEAN PATENT SPECIFICATION

(11) **EP 3 659 657 B1**
(45) Date of publication and mention of the grant of the patent: **21.10.2026**
(21) Application number: 19768652.0
(22) Date of filing: 05.03.2019
(51) Int. Cl.: A61M 5/32, A61M 5/00

(54) **INTRADERMAL NEEDLE, PACKAGED PRODUCT THEREOF, AND INJECTION DEVICE**
INTRADERMALE NADEL, VERPACKTES PRODUKT DAVON UND INJEKTIONSVORRICHTUNG
AIGUILLE INTRADERMIQUE, PRODUIT EMBALLÉ ASSOCIÉ ET DISPOSITIF D'INJECTION

(30) Priority: 16.03.2018 JP 2018049360
(43) Date of publication of application: 03.06.2020
(73) Proprietor: Terumo Kabushiki Kaisha, Tokyo 151-0072 (JP)
(72) Inventor: NAKAJIMA, Kentaro, Nakakoma-gun, Yamanashi 409-3853 (JP); IWASE, Yoichiro, Ashigarakami-gun, Kanagawa 259-0151 (JP)
(74) Representative: TBK
(86) International application number: PCT/JP2019/008668
(87) International publication number: WO 2019/176657

(56) References cited:
- WO-A1-2014/003632
- WO-A1-2016/020662
- WO-A1-2016/158144
- WO-A1-2016/158144
- WO-A1-2017/001925
- JP-A- 2015 525 605
- US-A- 5 919 165
- US-B1- 6 413 243

## Description

This invention relates to an intradermal needle used for injecting drug into living body, a packaging article thereof, and an injection device.

An intradermal needle has been proposed for injecting drug into an upper layer of skin. Since the intradermal needle is designed to keep a needle tip of a needle tube within the upper layer of skin, the needle tube has a length of protrusion of 3 mm or shorter. By virtue of such short length of protrusion of the needle tube, the intradermal needle is less likely to cause erroneous puncture, as compared with widely-used needles for hypodermic injection. Even the intradermal needle however remains to expose the needle tip, so that there remains a risk that the needle tip of the needle tube may accidentally puncture the user after the drag administration or upon disposal of the injection device.

Aiming at solving this problem, document WO 2016/158143 A1 describes a technique of providing a pivotable protector to the intradermal needle. With the intradermal needle in WO 2016/158143 A1, the needle tip can be prevented from accidentally puncturing the user, by pivoting the protector to cover the needle tip of the needle tube after use.

Document WO 2016/158144 A1 is considered as closest prior art. It discloses a similar drug injection device having a needle and flange like widened part at a needle hub. A pivot axis for a cover is provided at an edge of the widened part.

Document WO 2014/003632 A1 discloses another protective cover for disposable injection needles for subcutaneous injection. A pivot axis is perpendicular to a needle axis.

Document WO 2016/020662 A1 discloses a retractable needle assembly. By operating a lever, a protruding needle becomes retracted into a housing.

Document WO 2017/001925 discloses a single use delivery device prefilled with a reconstitutable agent. Here, a lid is arranged in a manner similar to that of WO 2016/158144 A1 (D1), but is provided with a lock hinge capable of maintaining an open or closed position of the lid.

Document US 6,413,243 B1 discloses a lengthy injection needle which can be covered by a pair of crocodile jaw like half covers acting in a synchronous manner.

### Summary of Invention

No special attention has, however, been paid for the aforementioned intradermal needle regarding convenience of housing in a packaging article, making the intradermal needle inconvenient to handle.

It is therefore the object of the present invention to provide an intradermal needle which may be housed more conveniently into a packaging article, may be improved to be easier to handle, a packaging article thereof, and an injection device.

The invention comprises an intradermal needle according to claim 1, a packaging article as defined in claim 12, and an injection device as defined in claim 13. According to an aspect of the present disclosure, there is provided an intradermal needle including a needle tube having a needle tip that can be punctured into a living body, a needle hub that supports the needle tube, a wide diameter part that extends like a flange from the needle hub, and a protector that is pivotable around an axis perpendicular to an axis of the needle hub, and is movable from an open position near the needle hub to a closed position where the needle tip is covered, and the protector having a pivoting axle part positioned on a base end side of the wide diameter part, and pivoting around the pivoting axle part.

According to the aforementioned intradermal needle, the pivoting axle part of the protector may be positioned so as not to overlap the wide diameter part which is the widest part of the intradermal needle, so that the protector may be sized compactly. Hence, the intradermal needle with the protector may now be housed within a small packaging article having an inner diameter comparable to the diameter of the wide diameter part.

In the intradermal needle, the axle of pivoting of the pivoting axle part may be arranged near the axis of the needle hub. With such structure, the protector can pivot nearly centered round the needle hub, making it possible to compactly house the lid, in the open position, more closely to the axis of the needle hub.

In the intradermal needle, the protector may have an arm that extends from the pivoting axle part, and a lid supported by the arm, wherein each of the pivoting axle part and the arm may be provided as a pair while placing the needle hub in between, and the lid may be supported by the pair of arms. With the lid thus supported by the pair of arms, the protector will have enhanced strength, and may be pivoted reliably around the axle of pivoting, even under force applied from directions other than the direction of pivoting, while preventing the arms and the lid from deforming.

In the intradermal needle, the wide diameter part may have formed therein a cutout side that extends in the direction of pivoting of the arms. With such structure, the wide diameter part is prevented from coming into contact with the arms in pivoting motion. Again in the intradermal needle, an interval between the one arm and the other arm may be set smaller than a diameter of the wide diameter part. This makes it possible to reduce the distance between the arms comparable to the width of the cutout side of the wide diameter part, to thereby downsize the protector, and to improve convenience for housing into the packaging article.

The intradermal needle may be provided with a restriction member that restricts pivoting of the protector in the open position and the closed position. This successfully prevents the lid of the protector from moving out of a predetermined range of pivoting, and from undesirably obstructing use of the intradermal needle.

In the intradermal needle, the pivoting axle part may have formed therein a resisting member that produces resistive force against pivoting of the protector. This successfully prevents the protector from unintentionally pivoting to obstruct use of the intradermal needle.

The intradermal needle may be provided with a lock mechanism that fixes the protector in the closed position. With this structure, the needle tip is prevented from being exposed, due to re-opening of the protector having been held in the closed position.

In the intradermal needle, the pivoting axle part may be arranged on a bracket mounted on the needle hub. This enables arrangement of the pivoting axle part near the needle hub, with a simple structure.

In the intradermal needle, a socket may be mounted so as to cover the wide diameter part from the base end side, and the pivoting axle part may be arranged on the socket. Further, the socket may have formed thereon a sliding face over which the protector slides, and the sliding face may be provided with a non-return mechanism that blocks reverse pivoting of the protector towards the open position. Further in the intradermal needle, the protector may be provided with a cutout formed by cutting out a part that falls, in the open position, within a predetermined range around the needle hub. With such socket mounted thereon, the strength will be enhanced since the arms of the protector are supported by the sliding face of the socket. In addition, the non-return mechanism can allow the protector to reliably pivot to the closed position.

According to another aspect of this invention, there is provided a packaging article of the intradermal needle, which includes the aforementioned intradermal needle, and a container that houses the intradermal needle, wherein the container has a bottom face that is formed to give an inner diameter nearly equal to the diameter of the wide diameter part of the intradermal needle, and the intradermal needle is housed in the container, with the protector kept in the open position. As described above, the intradermal needle of this invention has a protector that can be housed compactly, and the packaging article that houses them may use a container designed for intradermal needle having no protector.

According to still another aspect of this invention, there is provided an injection device having the aforementioned intradermal needle, and a syringe attached to the intradermal needle in a detachable manner. Hence, after use of the injection device, the protector of the intradermal needle may be pivoted to the closed position to cover the needle tip, so that the intradermal needle may be disposed safely, without inducing erroneous puncture.

With the intradermal needle, the packaging article thereof, and the injection device according to these aspects, convenience for housing in a small packaging article and convenience for handling will be improved.

### Brief Description of Drawings

Fig. 1 is a perspective view illustrating a packaging article housing an intradermal needle of a first embodiment of this invention.
Fig. 2 is a perspective view illustrating the intradermal needle according to the first embodiment of this invention, with a protector held in the open position.
Fig. 3 is an exploded perspective view illustrating the protector illustrated in Fig. 2.
Fig. 4 is a perspective view illustrating an injection device having the intradermal needle according to a first embodiment of this invention.
Fig. 5 is a cross-sectional view illustrating the intradermal needle illustrated in Fig. 2, with a syringe attached thereto.
Fig. 6 is a perspective view illustrating the intradermal needle illustrated in Fig. 2, with the protector held in the closed position.
Fig. 7 is a perspective view illustrating a packaging article housing an intradermal needle of a second embodiment of this invention.
Fig. 8 is a perspective view illustrating the intradermal needle according to the second embodiment of this invention, with a protector held in the open position.
Fig. 9 is an exploded perspective view illustrating the protector illustrated in Fig. 8.
Fig. 10 is a perspective view illustrating the intradermal needle illustrated in Fig. 8, with the protector held in the closed position.

### Description of Embodiments

Preferred embodiments of this invention will be detailed below, referring to the attached drawings.

### (First Embodiment)

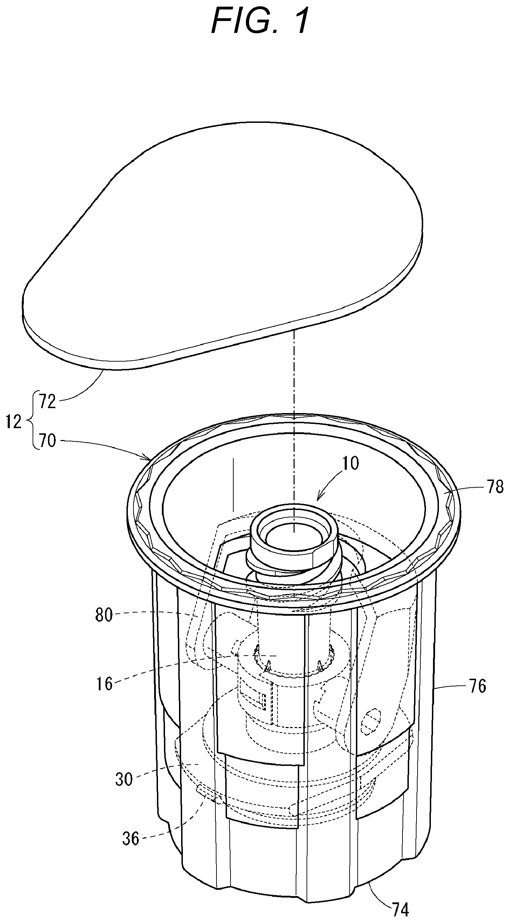

An intradermal needle 10 of this embodiment is provided as a product as illustrated in Fig. 1, while being housed in a packaging article 12 that individually packages the intradermal needle (medical needle) 10. The intradermal needle 10, sealed in the packaging article 12, is kept aseptic until just before use.

The packaging article 12 has a container 70 that houses the intradermal needle 10, and a seal member 72 that closes the container 70. When used, the seal member 72 is peeled off by the user from the container 70, thus making the user accessible to the intradermal needle 10 in the container 70.

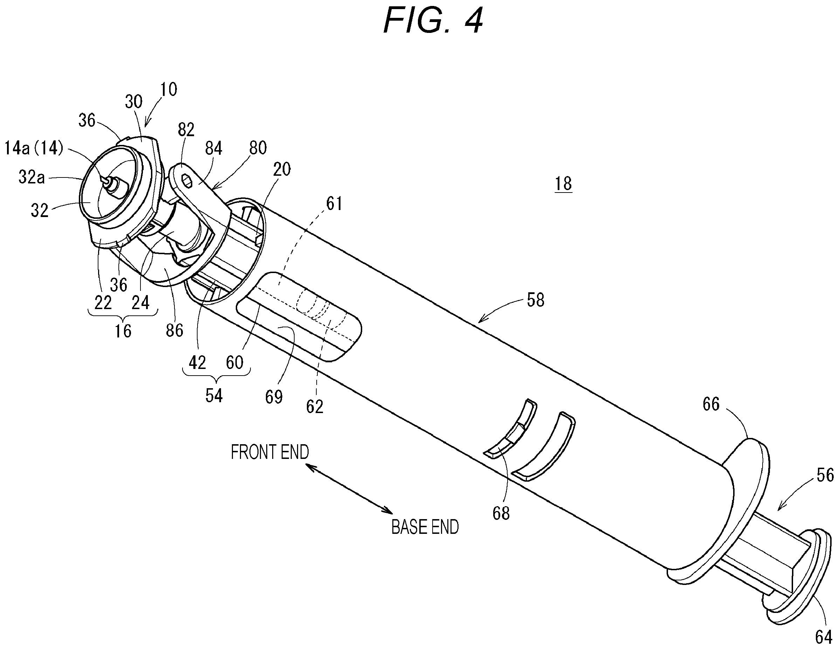

The container 70 is formed into a cylindrical shape, with an inner space formed therein, and a bottom face 74 thereof has a circular shape whose diameter is slightly larger than the diameter of the wide diameter part 30 of the needle hub 16. Also a cylinder wall 76 that rises up from the bottom face 74 in the axial direction of the intradermal needle 10 is formed to give an inner diameter slightly larger than the diameter of the wide diameter part 30. In the cylinder wall 76, an engagement part (not illustrated) that engages with a claw 36 of the wide diameter part 30 is formed. The engagement part and the claw 36 contribute to prevent the intradermal needle 10 from disengaging the container 70, and to restrict pivoting of the intradermal needle 10 around the axis, making it easy to attach a syringe 20 (see Fig. 4). On the top end of the cylinder wall 76, a flange 78, onto which a seal member 72 is bonded, is formed.

The intradermal needle 10 is housed in such packaging article 12, with the protector 80 held in the open position near the needle hub 16. Since the protector 80, in the open position, can fall within a cylindrical region having a diameter nearly equal to the diameter of the wide diameter part 30, so that the packaging article 12 sized according to the wide diameter part 30 can now house the intradermal needle 10.

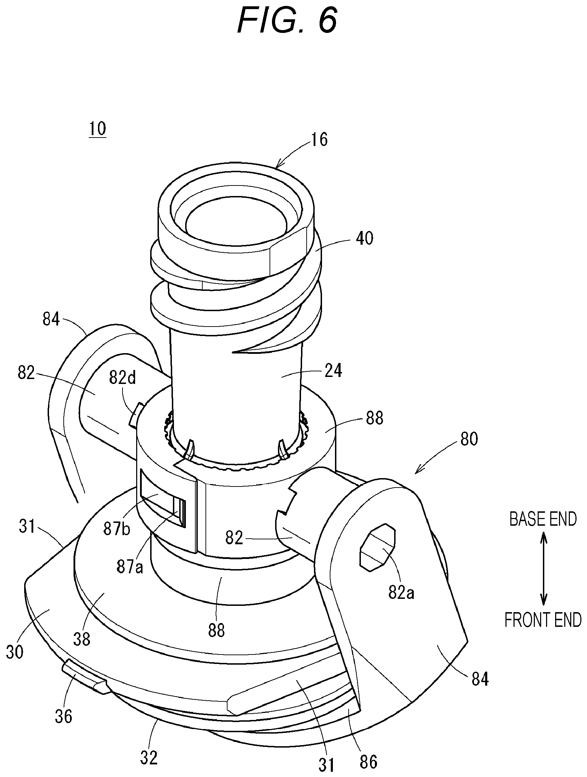

The intradermal needle 10 enclosed in the packaging article 12, with the protector 80 held in the open position, is used as it is in an injection device 18 (see Fig. 4). The intradermal needle 10 is designed to cover the needle tip 14a, after used, with the protector 80 by pivoting it around the axle of pivoting to the closed position as illustrated in Fig. 6.

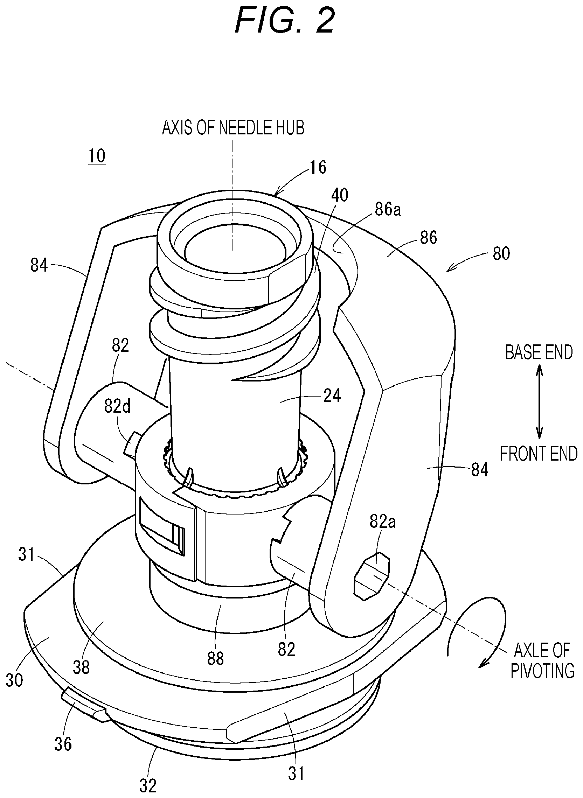

Next, a structure of the protector 80 provided to the intradermal needle 10 will be explained. The protector 80 has, as illustrated in Fig. 2, a pair of connection parts 82, an arm 84 that extends from each of the connection parts 82, a lid 86 supported by the pair of arms 84, and a bracket 88 mounted on a second member 24 of the needle hub 16. Now in the description below, the direction approaching the axis of the needle hub 16 is referred to as inner or inside, meanwhile the direction departing from the axis is referred to as outer or outside. Also note a face closer to the axis of the needle hub 16 is referred to as an inner circumferential face, meanwhile a face on the side departing from the axis is referred to as an outer circumferential face.

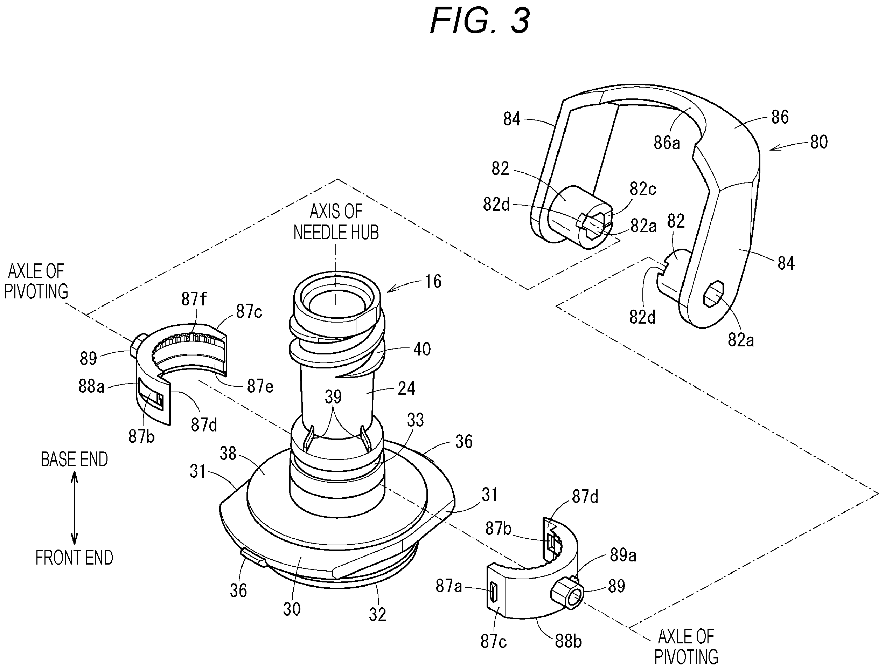

The bracket 88 is a cylindrical component that holds the second member 24 in between from the outer circumferential side. As illustrated in Fig. 3, the bracket 88 is composed of halves, which are a split piece 88a and a split piece 88b. Each of the split piece 88a and the split piece 88b has a connection part having a wedge 87c thinned towards the end, and a receiving part 87d that receives the wedge 87c. The wedge 87c is provided with a stopper piece 87a for preventing unintentional release, and a hole 87b that engages with the stopper piece 87a is formed in the receiving part 87d. The split pieces 88a, 88b are engaged by inserting the wedges 87c of the split pieces 88a, 88b into the receiving parts 87d, and by engaging the stopper pieces 87a with the holes 87b, thereby the bracket 88 is mounted so as to hold the second member 24 in between.

Each of the split pieces 88a, 88b has a ring-like protrusion 87e formed on the inner circumferential face, and a plurality of ribs 87f arranged at intervals in the circumferential direction. The protrusion 87e, when fitted in a groove 33 formed in a side wall of the second member 24, blocks the bracket 88 from moving towards the front end or the base end. With the bracket 88 mounted on the second member 24, the ribs 87f engage with pivoting stop protrusions 39 that extend from the outer circumference of the second member 24 in radial directions. Rotation of the bracket 88 around the axis of the needle hub 16 may thus be blocked by the pivoting stop protrusions 39 and the ribs 87f.

Each of the split pieces 88a, 88b has, on the outer circumferential face and at the center thereof in the circumferential direction, an axle pin 89 (pivoting axle part) formed so as to extend outwards. One axle pin 89 is arranged at an opposite position 180 degrees from the other axle pin 89 while placing the second member 24 in between, and the center axis of these axle pins 89 extends perpendicularly to the axis of the second member 24. The axle pins 89 are members that support the connection parts 82, whose center axis serves as an axle of pivoting of the protector 80. Note that the center axis (axle of pivoting or axis of rotation) of the axle pins 89 is not always necessarily perpendicular to the axis of the needle hub 16, and instead may be laid in the vicinity of the axis of the needle hub 16.

Each axle pin 89 is formed in a polygonal shape, and is made slidable against a hole 82a formed again in a polygonal shape. This produces resistive force that acts to obstruct pivoting motion of the protector 80, and blocks free pivoting of the protector 80. That is, the polygonal outer circumferential faces of the axle pins 89 and the polygonal holes 82a constitute the resisting member, by which the protector 80 is prevented from unintentionally pivoting.

The axle pin 89 has a restriction projection 89a that extends from the outer side face thereof. The restriction projection 89a engages with an open position restriction trench 82c and a closed position restriction trench 82d of each connection part 82 and can thereby stop the protector 80 in the open position or in the closed position. That is, the restriction projection 89a, the open position restriction trench 82c and the closed position restriction trench 82d constitute the restriction member.

In the protector 80, the connection part 82, the arm 84 and the lid 86 are formed integrally. Among them, each of the connection part 82 and the arm 84 is provided as a pair, and the lid 86 is supported from both sides by the pair of arms 84. The connection part 82 is provided as a pair, corresponded to the axle pins 89. Each connection part 82 is formed into a cylindrical shape that extends in the direction of axle of pivoting, and has at the center thereof a hole 82a that engages with each axle pin 89. The connection parts 82 are attached to the bracket 88, by fitting the connection parts 82 having the holes 82a on the axle pins 89 from outside. Each hole 82a has a polygonal inner circumferential face, so as to produce resistive force against the axle pin 89 in the direction of pivoting.

Each connection part 82 has the open position restriction trench 82c and the closed position restriction trench 82d formed at the inner end thereof. The open position restriction trench 82c engages with the restriction projection 89a of the axle pin 89, when the protector 80 is in the open position. This makes the protector 80 stop in the open position. That is, as a result of engagement of the restriction projection 89a with the open position restriction trench 82c, the protector 80 is restricted from pivoting towards the open position. Meanwhile, the closed position restriction trench 82d engages with the restriction projection 89a of the axle pin 89, when the protector 80 is pivoted to the closed position where the lid 86 covers the needle tip 14a of the needle tube 14 (see Fig. 6). That is, as a result of engagement of the restriction projection 89a with the closed position restriction trench 82d, the protector 80 is restricted from pivoting towards the closed position.

The arm 84 extends from the end of the outer circumference of the connection part 82, towards the outside of the axle of pivoting. The length of the arm 84 is determined to an extent that the lid 86 will not interfere the wide diameter part 30, when the protector 80 is pivoted to the closed position. In addition, the interval (distance in the direction of axle of pivoting) between the one arm 84 and the other arm 84 is determined smaller than the diameter of the wide diameter part 30 (but excluding the cutout side 31). With such structure, the protector 80 may be downsized, and the intradermal needle 10 may be housed within a cylindrical range having a diameter nearly equal to the diameter of the protector 80, with the protector 80 held in the open position.

The wide diameter part 30 has a pair of cutout sides 31 formed conforming to the interval between the arms 84. These cutout sides 31 are formed inside the inner face of the arms 84, and extend in the direction of pivoting of the arms 84. The wide diameter part 30 is thus designed not to obstruct pivoting of the arms 84.

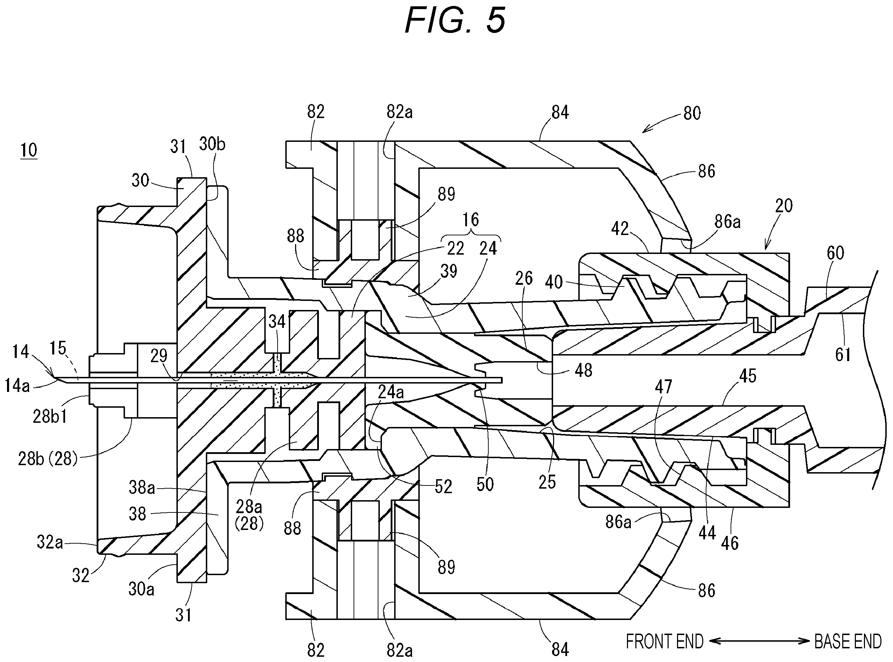

The lid 86 is formed so as to lie across the pair of arms 84. At the center of the lid 86, a cutout 86a is formed by performing semicircular cutting out operation. The cutout 86a is formed so as to eliminate a part of the lid 86 which comes near the axis of the second member 24 when the protector 80 is held in the open position. With the cutout 86a thus provided, there is produced, as illustrated in Fig. 2, a gap between the male screw 40 of the second member 24 and the lid 86, into which the front end of the syringe 20 (see Fig. 5) can be inserted. The lid 86 may alternatively be formed to give a curved face as illustrated in Fig. 3, without special limitation.

The aforementioned protector 80 may be attached according to the procedures below. First, there is provided the needle hub 16 whose wide diameter part 30 has preliminarily formed therein the cutout sides 31 and the groove 33. The split pieces 88a, 88b are then positioned in predetermined directions relative to the second member 24 of the needle hub 16, and mounted on the second member 24 so as to hold it from the outer circumferential side, to build up the bracket 88. Next, the connection part 82 is brought, with the arms 84 stretched apart, close to the axle pins 89 of the bracket 88, and the axle pins 89 are inserted into the holes 82a of the connection part 82, to build up the protector 80. The protector 80 is then suitably pivoted to engage the restriction projections 89a with the open position restriction trench 82c to thereby fix the protector 80 to the open position. Attachment of the protector 80 thus finishes. The thus manufactured intradermal needle 10 is enclosed in the packaging article 12 (see Fig. 1), and marketed as a product.

The injection device 18 having the intradermal needle 10 will be explained below, featuring an internal structure of the intradermal needle 10. The intradermal needle 10 has, as illustrated in Fig. 4, a needle tube 14 and a needle hub 16, and composes a component of an injection device 18. When used, the intradermal needle 10 is assembled on a syringe 20 provided separately from the intradermal needle 10. The user attaches the syringe 20 to the intradermal needle 10 to assemble the injection device 18, and then punctures a living body with the needle tip 14a of the intradermal needle 10. Upon pressing a plunger 56 of the syringe 20 while keeping a state of puncture, a chemical solution contained in the syringe 20 is intradermally injected through the needle tube 14 into the living body.

After injection, the intradermal needle 10 is disposed, with the used needle tube 14 covered and unexposed with the protector 80, thus making it possible to avoid accidental puncture with the needle tube 14. The individual components of the injection device 18 (intradermal needle 10 and syringe 20) will be explained below.

As illustrated in Fig. 5, the needle tube 14 of the intradermal needle 10 is given in the form of rigid hollow tube, with a needle hole 15 arranged at the axial center. At the frontmost part of the needle tube 14, the needle tip 14a having a sharp blade face. The needle tube 14 may have a thickness not specifically limited, whose gauge number is typically 26 to 33 (0.2 to 0.45 mm), and more preferably 30 to 33. Materials for composing the needle tube 14 are exemplified by stainless steel, aluminum, aluminum alloy, titanium, titanium alloy, other metals, and hard resins.

The needle hub 16 of the intradermal needle 10 has a first member 22 that fixes the needle tube 14, and a second member 24 on which the syringe 20 to be attached to the first member 22 is assembled. Materials for composing the first and second members 22, 24 are exemplified by resin materials such as polycarbonate, polypropylene, and polyethylene. The needle hub 16 also has an elastic member 26 inside the second member 24.

The first member 22 has an axial part 28 that directly holds the needle tube 14, the wide diameter part 30 that extends radially outward from the outer circumferential face of the axial part 28, and a ring protrusion 32 that extends ahead of an end face 30a of the wide diameter part 30. The axial part 28 is formed into a near cylindrical shape, and, at the axial center thereof, provided with a fixing hole 29 that houses and fixes the needle tube 14. With the needle tube 14 inserted into the fixing hole 29, an adhesive 34 is injected to fix the needle tube 14 and the first member 22.

The axial part 28 has a housing part 28a housed in the second member 24, and a protruding part 28b that protrudes ahead of the wide diameter part 30. The protruding part 28b of the axial part 28 protrudes slightly ahead of the ring protrusion 32, and has an end face 28b1 that comes into contact with a surface of a living body. The needle tube 14 fixed to the fixing hole 29 protrudes an appropriate dimension (length of protrusion) ahead of the end face 28b1. For example, the length of protrusion of the needle tube 14 is designed equivalent to the depth of dermis from the skin surface of the living body, which preferably falls in the range from approximately 0.5 to 3.0 mm.

The wide diameter part 30 is formed like a disk that extends from the outer circumferential face of the axial part 28 in the direction perpendicular to the center axis of the axial part 28. The wide diameter part 30 extends further beyond the ring protrusion 32 in the radial direction. On the base end face 30b, which is a face on the base end side of the wide diameter part 30 (a face opposite to the end face 30a having the ring protrusion 32 arranged thereon), the second member 24 is bonded. As illustrated in Fig. 4, on the outer circumference of the wide diameter part 30, a pair of claws 36 are formed so as to protrude outward. The pair of claws 36 are arranged on the opposite sides (180° degrees opposite position) on the outer circumference of the wide diameter part 30.

The ring protrusion 32 is a part that shortly protrudes like a wall from the wide diameter part 30 towards the front end, and is formed to give a cylindrical shape that surrounds the axial part 28 while keeping a predetermined distance in between. When using the intradermal needle 10, the entire range of an end face 32a of the ring protrusion 32 comes into contact with the skin of the living body. The ring protrusion 32 can therefore guide the injection device 18 so as to keep a posture of puncture perpendicularly to the skin, and can keep a constant depth of penetration of the needle tube 14 into the skin.

As illustrated in Fig. 5, the second member 24 is formed to give a near cylindrical shape with a through-hole 25 formed along the center axis. On the front end side of the through-hole 25, the housing part 28a of the first member 22 is inserted, meanwhile in the middle of the through-hole 25, an elastic member 26 is housed. On the base end side of the through-hole 25, a nozzle 44 of the syringe 20 is inserted during assemblage of the injection device 18. The inner circumferential face, on the base end side, of the through-hole 25 is tapered so as to allow surface contact with the outer circumferential face of the nozzle 44.

On the front end of the second member 24, there is provided a connecting wide diameter part 38 that extends outwards in the radial direction. The outer circumferential edge of the connecting wide diameter part 38 falls inside the outer circumferential edge of the wide diameter part 30 of the first member 22. The connecting wide diameter part 38 is fixed at its end face 38a to the base end face 30b of the wide diameter part 30, by an appropriate method of bonding such as vibration welding. On the outer circumferential face on the base end side of the second member 24, there is provided a male screw 40 on which a female screw 47 of the syringe 20 is screwed.

In other words, the syringe 20 has in its front end part 42, a nozzle 44 having an ejection channel 45 communicated with a reservoir 61 of the chemical liquid, and a connector 46 arranged around the nozzle 44, with the female screw 47 threaded on the inner face. With the male screw 40 and the female screw 47 engaged, the end face of the nozzle 44 comes into contact with, and pressurizes the base end face of the elastic member 26.

The elastic member 26 of the intradermal needle 10 is a cylindrical relay member that keeps the base end of the needle tube 14 liquid-tight, and holds the needle hole 15 opposing to the ejection channel 45 of the nozzle 44. In the elastic member 26, a needle tube hole 48 is provided, and in the needle tube hole 48, an inner projection 50 that holds the inserted needle tube 14 in contact therewith is formed. The elastic member 26 is tightly fixed while fitting itself to the inner circumference of the through-hole 25 of the second member 24, and while allowing an outward projection 52, which projects at the front end side outwards in the radial direction, to be caught between the base end face of the first member 22 and a step 24a of the second member 24.

The second member 24 has the bracket 88 mounted on the outer circumference thereof. The bracket 88 is provided with a pair of axle pins 89. The axle pins 89 are arranged at 180 degrees opposite positions on the outer circumference of the bracket 88. The axle pins 89 are individually fitted with the connection parts 82 having the holes 82a. Each connection part 82 protrudes outwards by a predetermined length. From each connection part 82, each arm 84 extends. The lid 86 is formed at free ends of such pair of arms 84. The lid 86 has the cutout 86a that produces, between the lid 86 in the open position and the needle hub 16, the gap into which the syringe 20 can be inserted. That is, provision of the cutout 86a enables attachment of the syringe 20 to the needle hub 16, without being interfered by the protector 80 even when the protector 80 is housed in the open position near the axis of the needle hub 16.

Meanwhile, the syringe 20 of the injection device 18 illustrated in Fig. 4 is given as a prefilled syringe having the chemical liquid preliminarily filled therein. The syringe 20 has a syringe body 54, a plunger 56 that is inserted into the syringe body 54 in a relatively movable manner, and a holder 58 that covers the outside of the syringe body 54.

The syringe body 54 has the front end part 42 (nozzle 44 and connector 46), and a barrel 60 having a reservoir 61 that communicates with the front end part 42 and stores the chemical liquid. Meanwhile, the plunger 56 has at the front end thereof a gasket 62 that is inserted liquid-tight into the reservoir 61, and has at the base end thereof an operation part 64 pushed by the user of the injection device 18. Alternatively, the syringe 20 may be of a type having the gasket 62 preliminarily housed in the reservoir 61, allowing the plunger 56 to be attached to the gasket 62 when the syringe 20 is used.

The holder 58 is a cylindrical body that houses and fixes the syringe body 54, and is used for thickening the injection device 18, making it more convenient for the user to grip. Hence the holder 58 has on the base end thereof a finger flange 66 on which fingers of the user who pushes the operation part 64 of the plunger 56 are hooked. The injection device 18 may alternatively have no holder 58.

The holder 58 has, arranged on the inner wall thereof surrounding the inner space, a plurality of support pieces (not illustrated) that support the front end part of the barrel 60 of the syringe body 54. The holder 58 also has, on the outer circumference thereof, a lock window 68 that locks a flange (not illustrated) provided to the base end of the syringe body 54, and the holder 58 also has at around the front end thereof a check window 69 through which the reservoir 61 of the syringe body 54 can be visually checked.

The intradermal needle 10, the packaging article 12, and the injection device 18 according to this embodiment are basically composed as described above. Operations of these items will be explained below.

The intradermal needle 10 is marketed as a product as illustrated in Fig. 1, while being enclosed in the container 70 of the packaging article 12. As described above, the intradermal needle 10 in this state has the protector 80 held in the open position near the axis of the needle hub 16. The packaging article 12 keeps the intradermal needle 10 air-tight, with the seal member 72 bonded to the flange 78 of the container 70.

When using the injection device 18, the seal member 72 of the packaging article 12 is peeled off, the front end of the syringe 20 is inserted into the packaging article 12, so as to connect the base end of the intradermal needle 10 and the syringe 20. That is, as illustrated in Fig. 4, the nozzle 44 of the front end part 42 of the syringe 20 is inserted into the through-hole 25 of the second member 24, and the female screw 47 of the connector 46 is screwed on the male screw 40 of the intradermal needle 10. In this process, the protector 80 of the intradermal needle 10 is kept in the open position as illustrated in Fig. 5. The connector 46 of the syringe 20 is inserted in the gap between the semicircular cutout 86a formed in the lid 86 of the protector 80 and the second member 24.

After the intradermal needle 10 and the syringe 20 are connected, the syringe 20 is pulled out from the packaging article 12. In this way, the pair of claws 36 of the intradermal needle 10 are released from the packaging article 12, and thereby the intradermal needle 10 and the syringe 20 are integrally taken out from the packaging article 12.

The needle tube 14 of the intradermal needle 10 is then punctured into the living body to inject the chemical liquid having been stored in the reservoir 61 of the syringe 20. The protector 80 in this state is held in the open position, while keeping the open position restriction trench 82c of the connection part 82 engaged with the restriction projection 89a. After injecting the chemical liquid, the used intradermal needle 10 is disposed.

When disposing the intradermal needle 10, the user pivots the protector 80 with fingers to the closed position. In this process, upon application of a predetermined level or larger force in the direction of pivoting of the protector 80 towards the closed position, the arms 84 will elastically deform outwards, and the restriction projections 89a are released from the open position restriction trenches 82c to thereby make the arms 84 freely pivotable. Upon further pivoting of the protector 80 towards the closed position, the restriction projections 89a engage with the closed position restriction trenches 82d (see Fig. 3) of the connection part 82, thus the protector 80 stops pivoting. The protector 80 thus moves to the closed position. In the closed position, the protector 80 is held so as not to escape the closed position, as a result of engagement of the restriction projections 89a with the closed position restriction trenches 82d of the fixed connection part 82. With the needle tip 14a of the needle tube 14 thus covered with the lid 86 of the protector 80, the user is protected from the sharp needle tip 14a. Thus the intradermal needle 10 can be disposed safely.

As described above, the intradermal needle 10 of this embodiment has the pivoting axle part (axle pin 89) of the protector 80 provided on the bracket 88 which is mounted on the base end side of the wide diameter part 30. This makes the protector 80 movable by pivoting nearly centered round the needle hub 16, and thereby the protector 80 may be housed compactly in the open position near the needle hub 16. This makes it possible to house the intradermal needle 10 with the protector 80 within the packaging article 12 whose inner diameter is nearly equal to the diameter of the wide diameter part 30.

The protector 80 is designed to include the pairs of connection parts 82 and the arms 84 arranged while placing the second member 24 in between, and to make the pair of arms 84 support the lid 86 from both sides. This enhances strength of the protector 80, makes it less likely to deform under force applied from directions other than direction of pivoting, and makes the protector 80 pivotable safely to the closed position.

In the wide diameter part 30 of the needle hub 16, the cutout sides 31 that extend in the direction of pivoting of the arms 84 are formed. With such structure, the wide diameter part 30 may be prevented from coming into contact with the arms 84 during pivoting operation of the arms 84. The interval between the pair of arms 84 may be made smaller than the diameter of the wide diameter part 30, which is variable depending on position of the cutout sides 31. This makes it possible to house the protector 80 with the arms 84 compactly in the open position, improving convenience for housing in a small packaging article 12.

The protector 80 is provided with the open position restriction trenches 82c, the closed position restriction trenches 82d, and the restriction projections 89a that engage with them, all of which constitute a restriction member for restricting pivoting at the open position and the closed position. This successfully restricts pivoting of the protector 80 enclosed in the packaging article 12, and prevents accidental contact of the lid 86 in the open position with the syringe 20. It also makes it possible to prevent the protector 80 in the closed position from pivoting, and allowing the needle tip 14a to accidentally expose.

With the cutout 86a in the lid 86 in a part closer to the axle of pivoting, formed so as to eliminate a part thereof near the axis of the second member 24, a space is now available for insertion of the syringe 20.

In the intradermal needle 10, the annular bracket 88 is mounted on the second member 24, and the axle pins 89 are arranged to the bracket 88. Hence the axle of pivoting of the protector 80 approaches near the axis of the needle hub 16, and the protector 80 in the open position can be housed near the needle hub 16, thus improving convenience for housing of the intradermal needle 10.

### (Second Embodiment)

An intradermal needle 10A of a second embodiment will be explained below. The inner structure of the needle hub 16 of the intradermal needle 10A is same as that of the intradermal needle 10 illustrated in Fig. 5, and will not therefore be illustrated redundantly. The structures in the intradermal needle 10A, same as those in the intradermal needle 10 having been described referring to Figs. 1 to 6, will be given same reference signs, and will not be detailed redundantly.

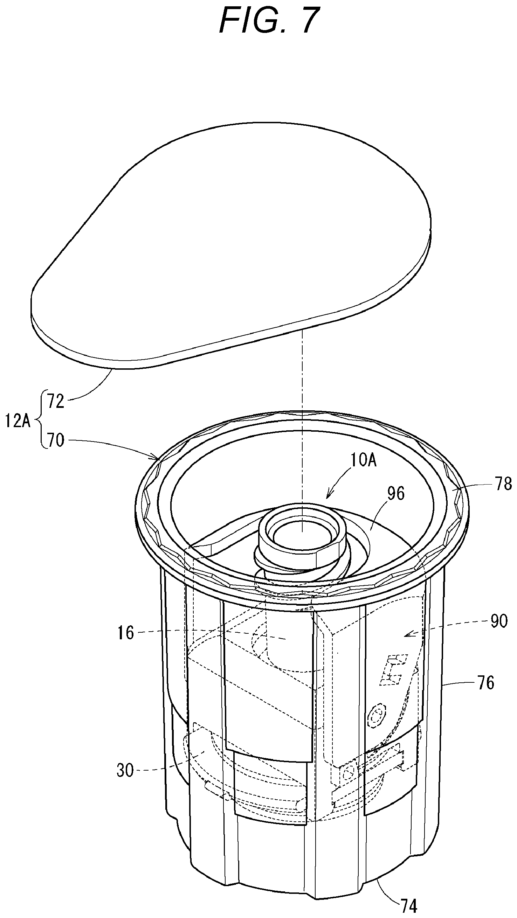

The intradermal needle 10A is marketed as a product enclosed in the packaging article 12A, with a lid 96 of a protector 90 held in the open position near the axis of the needle hub 16, as illustrated in Fig. 7. The packaging article 12A has a container 70 with a housing space for housing the intradermal needle 10A, and a seal member 72 that seals the container 70. The container 70 has a bottom face 74 whose inner diameter is nearly equal to the diameter of the wide diameter part 30 of the intradermal needle 10A, and from the bottom face 74, the cylindrical cylinder wall 76 extends in the axial direction of the intradermal needle 10A. The cylinder wall 76 has an inner diameter nearly equal to the diameter of the wide diameter part 30. The cylinder wall 76 has the flange 78 at the top end thereof, and the seal member 72 is bonded to the flange 78.

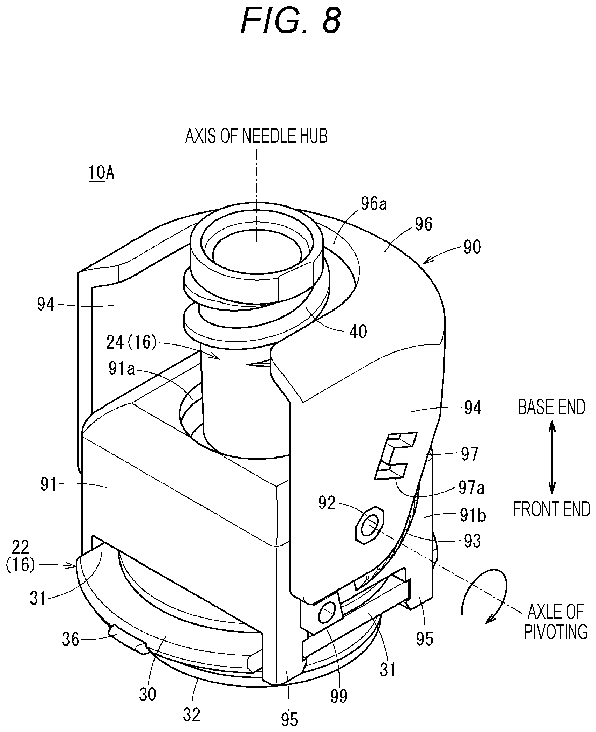

The intradermal needle 10A of this embodiment is composed of, as illustrated in Fig. 8, the needle hub 16 having the first member 22 and the second member 24, and the protector 90 mounted thereon. The protector 90 has a socket 91 mounted so as to cover the wide diameter part 30 from the base end side, the pair of arms 94 attached to the socket 91, and the lid 96 supported by the arms 94.

The protector 90 of this embodiment has the axle of pivoting positioned on the base end side of the wide diameter part 30, and the axle of pivoting extends in the direction perpendicular to the axis of the needle hub 16. The axle of pivoting of the protector 90 preferably lies perpendicular to the axis of the needle hub 16, or positioned so as to lie near the axis of the needle hub 16. This enables the protector 90 to pivot nearly centered round the needle hub 16, and thereby the lid 96 in the open position may be housed compactly near the needle hub 16.

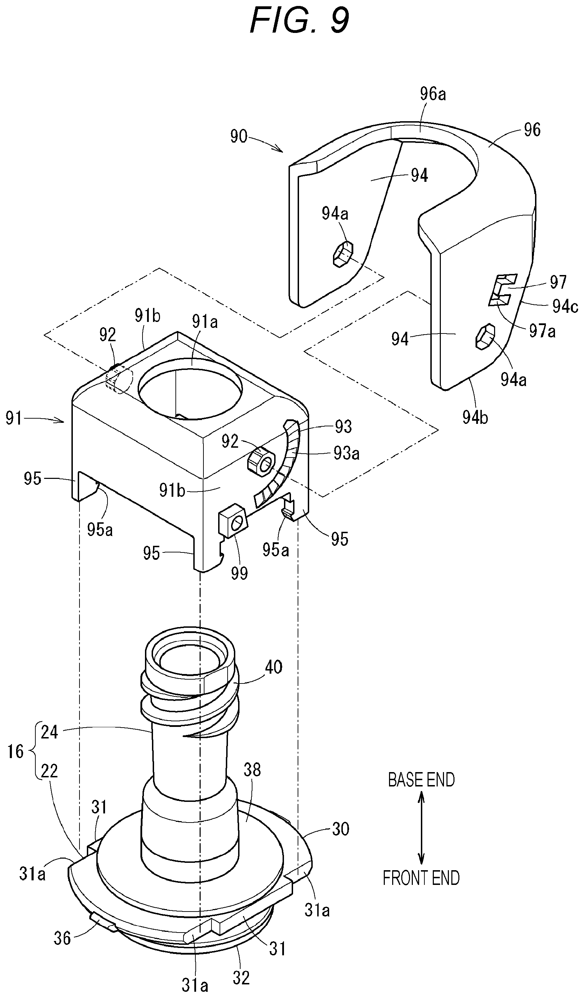

The socket 91 of the intradermal needle 10A is mounted on the wide diameter part 30 of the first member 22. The wide diameter part 30 has the pair of cutout sides 31 formed so as to straightly cutting out a part thereof in the direction of pivoting of the protector 90. These cutout sides 31 are formed on the pivoting face 91b of the socket 91 in a co-plane manner, so as not to interfere pivoting of the arms 94. As illustrated in Fig. 9, on both ends of cutout side 31, recesses 31a on which legs 95 of the socket 91 are engaged are formed. The legs 95 of the socket 91 engage their hooks 95a with the recesses 31a, to thereby mount the socket 91 on the wide diameter part 30.

The socket 91 is shaped like a box with an open end on the front end side, and has the legs 95 extended from four corners thereof on the front end side. Each leg 95 has the hook 95a that engages with the wide diameter part 30. By fitting the legs 95 with the recesses 31a of the wide diameter part 30 to thereby engage the hooks 95a with the wide diameter part 30 as described above, the socket 91 is fixed on the needle hub 16. The socket 91 also has a circular opening 91a formed in a face thereof on the base end side. The opening 91a is formed larger than the outer diameter of the second member 24 of the needle hub 16. With the second member 24 allowed to pass through the opening 91a, the socket 91 is mounted on the wide diameter part 30 without coming into contact with the sidewall of the second member 24.

Among from four side faces of the socket 91, a pair of pivoting faces 91b that are opposed to the arms 94 individually have the axle pins 92 that compose the pivoting axle part. Each axle pin 92 extends like a pillar in the direction perpendicular to the axis of the needle hub 16, whose center axis serves as the axle of pivoting of the protector 90. The axle pin 92 has a polygonal shape when viewed from the direction of axle of pivoting, and is designed to produce a predetermined level of resistive force against pivoting of the arms 94. Such axle pin 92 is also provided to the pivoting face 91b on the far side of the sheet of drawing, and these axle pins 92 are arranged on the same axle of pivoting.

The pivoting face 91b also has an arcuate ratchet groove 93 centered round the axle of pivoting, and a protrusion part 99 arranged on an extension ahead of one end of the ratchet groove 93. The ratchet groove 93 is a part engaged with the claw 97 of the arm 94, and the claw 97 is designed to move, as the arm 94 pivots, along the ratchet groove 93 while keeping contact therewith towards the protrusion part 99. In the ratchet groove 93, a plurality of dents 93a are formed. These dents 93a are formed to give a saw-like cross section with upright faces on the side closer to the protrusion part 99.

The protrusion part 99 also functions as the open position restriction member and the closed position restriction member. That is, upon contact of the protrusion part 99 with a first side edge 94b of the arm 94, the arm 94 is restricted from pivoting towards the open position. Meanwhile, upon contact of the protrusion part 99 with a second side edge 94c of the arm 94, the arm 94 is restricted from pivoting towards the closed position. The ratchet groove 93 and the protrusion part 99 may alternatively be arranged on both of the pair of pivoting faces 91b, but may be arranged only on one face as illustrated above.

The arms 94 and the lid 96 attached to the socket 91 are formed integrally. The arms 94 are provided as a pair corresponding to the axle pins 92, while being spaced from each other in the direction of axle of pivoting. The arms 94 are formed like a plate which is flat in the direction of axle of pivoting, and can pivot with the inner faces thereof slid over the pivoting faces 91b of the socket 91. In each arm 94, a hole 94a that engages with the axle pin 92 is formed. The hole 94a is formed to give a polygonal shape conforming to the axle pin 92. The arm 94 also has a clawed opening 97a in which the claw 97 is arranged. The claw 97 composes a non-return mechanism that engages with the dents 93a of the ratchet groove 93 to allow pivoting from the open position to the closed position, while prohibiting the reverse pivoting.

The lid 96 is a plate like member formed so as to lie across the pair of arms 94, with both side ends supported by the arms 94. Although not specifically limited, the lid 96 may alternatively be formed into a curved face as illustrated here. The lid 96 has at the center thereof a U-shaped cutout 96a. The cutout 96a is formed by cutting out a part of the lid 96, which comes near the axis of the needle hub 16 when the protector 90 is held in the open position as illustrated in Fig. 8. Provision of such cutout 96a produces a gap necessary for attaching the syringe 20, between the needle hub 16 and the lid 96. With the cutout 96a thus having the U shape which is oblong in the direction pivoting, the lid 96 can be pivoted to the closed position without interfering with the syringe 20, even when the syringe 20 is attached.

The socket 91 can be mounted on the protector 90 by aligning the legs 95 to the recesses 31a of the wide diameter part 30, and pressing them towards the wide diameter part 30. This allows the hooks 95a of the legs 95 to engage with the wide diameter part 30, to thereby fix the socket 91 on the wide diameter part 30. A molded article having the arms 94 and the lid 96 integrally molded therein is prepared, and the axle pins 92 are engaged with the holes 94a while keeping the arms 94 stretched apart, thus attaching the arms 94 and the lid 96 to the socket 91. The protector 90 is thus assembled to the needle hub 16.

The intradermal needle 10A of this embodiment is basically built as described above. Operations of these items will be explained below.

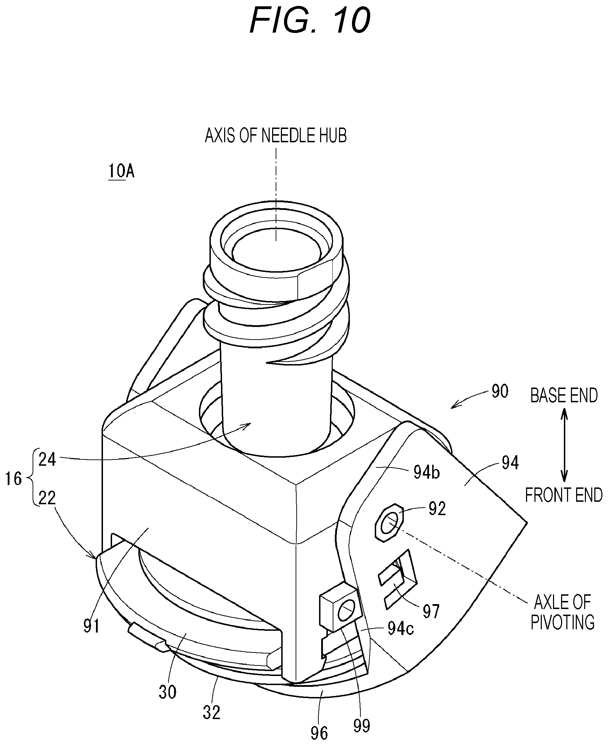

The intradermal needle 10A is marketed as a product while being enclosed in the packaging article 12A. The intradermal needle 10A is enclosed in the packaging article 12A with the protector 90 held in the open position, and becomes accessible to the user after the seal member 72 is peeled off from the container 70. The syringe 20 (see Fig. 4) is then assembled to the intradermal needle 10A. The protector 90 in this process is kept in the open position, with the aid of the resistive force in the direction of pivoting produced by the polygonal axle pins 92 (pivoting axle part) and the holes 94a. The protector 90 is kept in the open position also during use. After using the intradermal needle 10A, the user pivots the protector 90 with fingers to move the protector 90 to the closed position, as illustrated in Fig. 10.

The arms 94 of the protector 90 in this process pivot while keeping contact between the claw 97 of the arm 94 and the ratchet groove 93. The dents 93a of the ratchet groove 93 have the upright faces on the side closer to the closed position, which allows pivoting towards the closed position only, while restricting pivoting towards the open position. The arms 94 stop pivoting upon contact with the protrusion part 99 in the closed position. Hence the arms 94 are restricted by the ratchet groove 93 and the claw 97, and thereby the protector 90 is kept locked in the closed position. That is, the protector 90 in the intradermal needle 10A is fixed in the closed position, since the ratchet groove 93 and the claw 97 act as a lock mechanism. The needle tip 14a is thus kept covered with the lid 96, making the intradermal needle 10A safely disposable.

In the aforementioned intradermal needle 10A, the axle pins 92 (pivoting axle part) are provided to the socket 91 mounted on the wide diameter part 30. This makes it possible to arrange the axle of pivoting of the axle pins 92 (pivoting axle part) on the base end side of the wide diameter part 30, so as to lie near the needle hub 16, with minimum working on the needle hub 16. In addition, since the protector 90 is designed to pivot centered round the axle of pivoting near the needle hub 16, so that the lid 96 of the protector 90 in the open position may be housed near the axis of the needle hub 16, thus improving convenience for housing of the intradermal needle 10A.

In the intradermal needle 10A, the pair of axle pins 92 are provided to a box-like socket 91, and the pair of arms 94 are provided corresponding to the individual axle pins 92. Since a part of the arm 94 near the axle of pivoting is supported by the box-like socket 91, so that the protector 90 will have further improved strength. Hence the protector 90 may be moved reliably to the closed position while preventing the arms 94 and the lid 96 from deforming, even under force applied from directions other than the direction of pivoting.

In the intradermal needle 10A, since the wide diameter part 30 is provided with the cutout sides 31 that extend in the direction of pivoting of the arms 94, so that when the arms 94 is pivoted, the arms 94 are prevented from coming into contact with the wide diameter part 30. In addition, in the intradermal needle 10A, with the interval between the pair of arms 94 set smaller than the diameter of the wide diameter part 30, the protector 90 may be downsized, thus improving convenience for housing in the packaging article 12A.

In the intradermal needle 10A, the polygonal shape of the axle pins 92 contributes to produce resistive force against pivoting of the protector 90. This successfully prevents the protector 90 from unintentionally pivoting to obstruct use of the intradermal needle 10A.

In the intradermal needle 10A, the ratchet groove 93 is provided to the socket 91, and the claw 97 that comes into contact with the ratchet groove 93 is provided to the arm 94. This successfully blocks the protector 90 from pivoting in the reverse direction, and prevents the needle tip 14a from being exposed, due to re-opening of the protector 90 having been held in the closed position.

## Claims

1. An intradermal needle comprising:
a needle tube (14) having a needle tip (14a) that can be punctured into a living body; and
a needle hub (16), wherein the needle hub (16) includes a first member (22) that fixes the needle tube (14), and a second member (24) that is bonded to a proximal end side of the first member (22) and to which a syringe may be assembled, and a wide diameter part (30) located distally of the second member (24) and that extends like a flange from the first member (22); and
a protector (80, 90) that is pivotable around an axis perpendicular to an axis of the needle hub (16), **characterized in that** the protector is movable from an open position near the needle hub (16) to a closed position where the needle tip (14a) is covered by the protector (80, 90), and
the protector (80, 90) having a pivoting axle part (89) positioned on a base end side of the wide diameter part (30), and pivoting around the pivoting axle part, wherein the axle of pivoting of the pivoting axle part is arranged near or intersects the axis of the needle hub (16).

2. The intradermal needle according to claim 1, wherein the protector (80, 90) has an arm (84, 94) that extends from the pivoting axle part, and a lid (86, 96) supported by the arm (84, 94), each of the pivoting axle part and the arm (84, 94) is provided as a pair while placing the needle hub (16) in between, and the lid (86, 96) is supported by the pair of arms (84, 94).

3. The intradermal needle according to claim 2, wherein a distance from the one arm (84, 94) to the other arm (84, 94) is smaller than a diameter of the wide diameter part (30).

4. The intradermal needle according to claim 3, wherein a cutout side (31) that extends in a direction of pivoting of the arms (84, 94) is formed in the wide diameter part (30).

5. The intradermal needle according to claim 1, further comprising a restriction member configured to restrict pivoting of the protector (80, 90) in the open position and the closed position.

6. The intradermal needle according to claim 1, wherein a resisting member that is configured to produce resistive force against pivoting of the protector (80, 90) is formed in the pivoting axle part.

7. The intradermal needle according to claim 1, further comprising a lock mechanism that is configured to fix the protector (80, 90) to the closed position.

8. The intradermal needle according to claim 1, further comprising a bracket (88) mounted on the needle hub (16), and the pivoting axle part is arranged on the bracket (88).

9. The intradermal needle according to claim 1, further comprising a socket (91) mounted so as to cover the wide diameter part (30) from the base end side, and the pivoting axle part is arranged on the socket (91).

10. The intradermal needle according to claim 9, wherein the socket (91) has a sliding face over which the protector (90) is configured to slide, and the sliding face is provided with a non-return mechanism that is configured to block reverse pivoting of the protector (90) towards the open position.

11. The intradermal needle according to claim 1, wherein the protector (80, 90) is provided with a cutout (86a, 96a) formed by cutting out a part that falls, in the open position, within a predetermined range around the needle hub (16).

12. A packaging article (12, 12A) comprising:
a container (70) that houses
the intradermal needle (10, 10A) according to claim 1, and
the container (70) having a bottom face (74) of the container (70) that is formed to give an inner diameter nearly equal to a diameter of the wide diameter part (30), and housing the intradermal needle (10, 10A) with the protector (80, 90) kept in the open position.

13. An injection device comprising:
a syringe (20) attached to the second member (24) of an intradermal needle (10, 10A) according to claim 1 in a detachable manner.

## Patentansprüche

1. Intradermale Nadel, umfassend:
ein Nadelrohr (14) mit einer Nadelspitze (14a), die in einen lebenden Körper gestochen werden kann; und
einen Nadelanschluss (16), wobei
der Nadelanschluss (16) ein erstes Element (22) umfasst, das das Nadelrohr (14) fixiert, sowie ein zweites Element (24), das mit einer proximalen Endseite des ersten Elements (22) verbunden ist und an dem eine Spritze angebracht werden kann, und einen Abschnitt (30) mit großem Durchmesser, der distal zum zweiten Element (24) angeordnet ist und sich wie ein Flansch vom ersten Element (22) erstreckt; sowie
eine Schutzvorrichtung (80, 90), die um eine Achse senkrecht zur Achse der Nadelhülse (16) schwenkbar ist,
**dadurch gekennzeichnet, dass** die Schutzvorrichtung von einer offenen Position in der Nähe des Nadelanschlusses (16) in eine geschlossene Position bewegbar ist, in der die Nadelspitze (14a) von der Schutzvorrichtung (80, 90) abgedeckt ist, und
wobei die Schutzvorrichtung (80, 90) einen Schwenkachsenabschnitt (89) aufweist, der an einer Basisendseite des Abschnitts mit großem Durchmesser (30) angeordnet ist, und um den Schwenkachsenabschnitt schwenkbar ist, wobei die Schwenkachse des Schwenkachsenabschnitts nahe der Achse des Nadelanschlusses (16) angeordnet ist oder diese schneidet.

2. Intradermale Nadel nach Anspruch 1, wobei die Schutzvorrichtung (80, 90) einen Arm (84, 94), der sich vom Schwenkachsenabschnitt erstreckt, und einen vom Arm (84, 94) getragenen Deckel (86, 96) aufweist, wobei der Schwenkachsenabschnitt und der Arm (84, 94) jeweils paarweise ausgebildet sind, wobei der Nadelanschluss (16) dazwischen angeordnet ist, und der Deckel (86, 96) von dem Paar Arme (84, 94) getragen ist.

3. Intradermale Nadel nach Anspruch 2, wobei der Abstand zwischen dem einen Arm (84, 94) und dem anderen Arm (84, 94) kleiner ist als der Durchmesser des Abschnitts mit großem Durchmesser (30).

4. Intradermale Nadel nach Anspruch 3, wobei im Abschnitt (30) mit großem Durchmesser eine Aussparungsseite (31) ausgebildet ist, die sich in Schwenkrichtung der Arme (84, 94) erstreckt.

5. Intradermale Nadel nach Anspruch 1, die ferner ein Begrenzungselement umfasst, das so ausgebildet ist, dass es das Schwenken der Schutzvorrichtung (80, 90) in der offenen Position und in der geschlossenen Position begrenzt.

6. Intradermale Nadel nach Anspruch 1, wobei im Schwenkachsenabschnitt ein Widerstandselement ausgebildet ist, das so ausgebildet ist, dass es eine Widerstandskraft gegen das Schwenken der Schutzvorrichtung (80, 90) erzeugt.

7. Intradermale Nadel nach Anspruch 1, die ferner einen Verriegelungsmechanismus umfasst, der so ausgelegt ist, dass er die Schutzvorrichtung (80, 90) in der geschlossenen Position fixiert.

8. Intradermale Nadel nach Anspruch 1, die ferner eine an dem Nadelanschluss (16) angebrachte Klammer (88) umfasst, wobei der Schwenkachsenabschnitt an der Klammer (88) angeordnet ist.

9. Intradermale Nadel nach Anspruch 1, ferner mit einer Hülse (91), die so angebracht ist, dass sie den Abschnitt (30) mit großem Durchmesser von der Basisendseite her abdeckt, und wobei der Schwenkachsenabschnitt an der Hülse (91) angeordnet ist.

10. Intradermale Nadel nach Anspruch 10, wobei die Hülse (91) eine Gleitfläche aufweist, über die die Schutzvorrichtung (90) gleiten kann, und die Gleitfläche mit einem Rücklaufsperrmechanismus versehen ist, der so ausgelegt ist, dass er ein Zurückdrehen der Schutzvorrichtung (90) in Richtung der offenen Position verhindert.

11. Intradermale Nadel nach Anspruch 1, wobei die Schutzvorrichtung (80, 90) mit einer Aussparung (86a, 96a) versehen ist, die durch Ausschneiden eines Teils ausgebildet ist, der in der geöffneten Position innerhalb eines vorbestimmten Bereichs um den Nadelanschluss (16) liegt.

12. Verpackungsartikel (12, 12A), umfassend:
einen Behälter (70), der die intradermale Nadel (10, 10A) gemäß Anspruch 1 aufnimmt, und
wobei der Behälter (70) eine Bodenfläche (74) aufweist, die so ausgebildet ist, dass sie einen Innendurchmesser aufweist, der nahezu dem Durchmesser des Abschnitts (30) mit großem Durchmesser entspricht, und die intradermale Nadel (10, 10A) so aufnimmt, dass der Schutz (80, 90) in der geöffneten Position gehalten wird.

13. Eine Injektionsvorrichtung, umfassend:
eine Spritze (20), die abnehmbar an dem zweiten Element (24) einer intradermalen Nadel (10, 10A) gemäß Anspruch 1 befestigt ist.

## Revendications

1. Aiguille intradermique, comprenant :
un tube d'aiguille (14) ayant une pointe d'aiguille (14a) qui peut être utilisée pour effectuer une ponction dans un corps vivant ; et
un pavillon d'aiguille (16), dans laquelle le pavillon d'aiguille (16) inclut un premier élément (22) qui fixe le tube d'aiguille (14), et un second élément (24) qui est lié à un côté extrémité proximale du premier élément (22) et avec lequel une seringue peut être assemblée, et une partie de diamètre large (30) située distalement du second élément (24) et qui s'étend comme une bride depuis le premier élément (22) ; et
un organe de protection (80, 90) qui peut pivoter autour d'un axe perpendiculaire à un axe du pavillon d'aiguille (16), **caractérisée en ce que** l'organe de protection est mobile depuis une position ouverte près du pavillon d'aiguille (16) jusqu'à une position fermée où la pointe d'aiguille (14a) est couverte par l'organe de protection (80, 90), et
l'organe de protection (80, 90) ayant une partie à tourillon de pivotement (89) positionnée sur un côté extrémité de base de la partie de diamètre large (30), et pivotant autour de la partie à tourillon de pivotement, dans laquelle le tourillon de pivotement de la partie à tourillon de pivotement est agencé près de l'axe, ou l'intersecte, du pavillon d'aiguille (16).

2. Aiguille intradermique selon la revendication 1, dans laquelle l'organe de protection (80, 90) a un bras (84, 94) qui s'étend depuis la partie à tourillon de pivotement, et un couvercle (86, 96) supporté par le bras (84, 94), chacun de la partie à tourillon de pivotement et du bras (84, 94) est prévu sous forme de paire tout en plaçant le pavillon d'aiguille (16) entre ceux-ci, et le couvercle (86, 96) est supporté par la paire de bras (84, 94).

3. Aiguille intradermique selon la revendication 2, dans laquelle une distance depuis l'un bras (84, 94) jusqu'à l'autre bras (84, 94) est plus petite qu'un diamètre de la partie de diamètre large (30).

4. Aiguille intradermique selon la revendication 3, dans laquelle un côté découpe (31) qui s'étend dans une direction de pivotement des bras (84, 94) est formé dans la partie de diamètre large (30).

5. Aiguille intradermique selon la revendication 1, comprenant en outre un élément de restriction configuré pour restreindre le pivotement de l'organe de protection (80, 90) dans la position ouverte et la position fermée.

6. Aiguille intradermique selon la revendication 1, dans laquelle un élément résistant qui est configuré pour produire une force de résistance contre le pivotement de l'organe de protection (80, 90) est formé dans la partie à tourillon de pivotement.

7. Aiguille intradermique selon la revendication 1, comprenant en outre un mécanisme de verrouillage qui est configuré pour fixer l'organe de protection (80, 90) à la position fermée.

8. Aiguille intradermique selon la revendication 1, comprenant en outre un support (88) monté sur le pavillon d'aiguille (16), et la partie à tourillon de pivotement est agencée sur le support (88).

9. Aiguille intradermique selon la revendication 1, comprenant en outre une douille (91) montée afin de couvrir la partie de diamètre large (30) depuis le côté extrémité de base, et la partie à tourillon de pivotement est agencée sur la douille (91).

10. Aiguille intradermique selon la revendication 9, dans laquelle la douille (91) a une face de coulissement par-dessus laquelle l'organe de protection (90) est configuré pour coulisser, et la face de coulissement est pourvue d'un mécanisme de non-retour qui est configuré pour bloquer le pivotement inverse de l'organe de protection (90) vers la position ouverte.

11. Aiguille intradermique selon la revendication 1, dans laquelle l'organe de protection (80, 90) est pourvu d'une découpe (86a, 96a) formée en découpant une partie qui se trouve, dans la position ouverte, dans les limites d'une étendue prédéterminée autour du pavillon d'aiguille (16).

12. Article d'emballage (12, 12A), comprenant :
un contenant (70) qui loge
l'aiguille intradermique (10, 10A) selon la revendication 1, et
le contenant (70) ayant une face de fond (74) du contenant (70) qui est formée pour donner un diamètre intérieur pratiquement égal à un diamètre de la partie de diamètre large (30), et logeant l'aiguille intradermique (10, 10A) avec l'organe de protection (80, 90) maintenu dans la position ouverte.

13. Dispositif d'injection, comprenant :
une seringue (20) attaché au second élément (24) d'une aiguille intradermique (10, 10A) selon la revendication 1 de manière détachable.
